# EUROPÄISCHE PATENTSCHRIFT

(11) **EP 1 417 287 B1**
(45) Veröffentlichungstag und Bekanntmachung des Hinweises auf die Patenterteilung: **12.12.2012**
(21) Anmeldenummer: 01915150.5
(22) Anmeldetag: 19.01.2001
(51) Int. Cl.: C10M 129/68, C10M 105/32, C07C 67/04

(54) **ACYLOXYLIERTE FETTSÄURE ODER FETTSÄUREDERIVATE , INSBESONDERE SCHMIERSTOFFE UND DRUCKÜBERTRAGUNGSMITTEL, DEREN HERSTELLUNG UND VERWENDUNG**
NOVEL COMPOSITIONS CONTAINING ACETOXYLATED FATTY ACIDS OR FATTY ACID DERIVATES, IN PARTICULAR LUBRICANT AND PRESSURE TRANSMITTING COMPOSITIONS, THE PREPARATION AND USE THEREOF
NOUVELLES COMPOSITIONS DE MATIERES CONTENANT DES ACIDES GRAS ACETOYLES, EN PARTICULIER LUBRIFIANTS ET AGENTS DE TRANSMISSION DE PRESSION, LEUR PRODUCTION ET LEUR UTILISATION

(30) Priorität: 21.01.2000 DE 10002515; 21.01.2000 DE 10002516; 24.03.2000 DE 10014922
(43) Veröffentlichungstag der Anmeldung: 12.05.2004
(73) Patentinhaber: Fuchs Petrolub AG, 68169 Mannheim (DE)
(72) Erfinder: HÖLDERICH, Wolfgang, 67227 Frankenthal (DE); KELLER, Ulrich, 52078 Aachen (DE); FISCHER, Jutta, 51373 Leverkusen (DE); WECKES, Patrick, 47877 Willich (DE); MANG, Theo, 69469 Weinheim (DE); LUTHER, Rolf, 67346 Speyer (DE); WAGNER, Helena, 50737 Köln (DE)
(74) Vertreter: Müller Schupfner & Partner
(86) Internationale Anmeldenummer: PCT/EP2001/000594
(87) Internationale Veröffentlichungsnummer: WO 2001/053438

(56) Entgegenhaltungen:
- US-A- 2 115 341
- US-A- 3 676 500
- US-A- 4 709 097
- BLACK, L.T. ET AL: "Acetoxylation of methyl oleate with a resin catalyst" J. AMER. OIL CHEM. SOC., Bd. 44, Nr. 5, 1967, Seiten 310-312, XP001000539 in der Anmeldung erwähnt
- TULLOCH, A.P.: "Carbon-13 NMR Spectra of all the Isomeric Methyl Hydroxy- and Acetoxyoctadecanoates " ORG. MAGN. RESON., Bd. 11, Nr. 3, 1978, Seiten 109-15, XP001000352
- DATABASE WPI Section Ch, Week 199747 Derwent Publications Ltd., London, GB; Class D23, AN 1997-508813 XP002168558 & JP 09 241210 A (NEW JAPAN CHEM CO LTD), 16. September 1997 (1997-09-16)

## Beschreibung

Die vorliegende Anmeldung betrifft Stoffzusammensetzungen, die als Schmierstoffe und Druckübertragungsmittel geeignet sind, bestehend aus Estern von Fettsäuren, von Fettsäurederivaten und von deren Gemischen sowie ein Verfahren zur Herstellung dieser erfindungsgemäßen Ester durch Addition von Carbonsäuren und/oder Carbonsäureanhydriden und/oder Carbonsäurehalogeniden an die Doppelbindung und/oder Doppelbindungen von Fettsäuren, Fettsäurederivaten oder deren Gemischen sowie die Verwendung dieser erfindungsgemäßen Ester als umweltverträgliche, biologisch leicht abbaubare Schmierstoffe, Schmierstoffkomponenten, Schmierstoffadditive, Hydrauliköle, Druckübertragungsmittel, Getriebeöle und Funktionsflüssigkeiten.

### Stand der Technik

Allein in Deutschland gelangen jährlich etwa 520.000 t Schmierstoffe über Leckagen an Dichtungen oder Schläuchen, bei Reparaturarbeiten, in Ölunfällen oder durch Verdampfung und Vernebelung in die Umwelt. Durch Verwendung nachwachsender Rohstoffe, direkt oder in Form von deren Umwandlungsprodukten, können nicht nur fossile Rohstoffe eingespart, sondern auch die C0₂-Bilanz ausgeglichen sowie Flächenstillegungen zur Verminderung landwirtschaftlicher Überproduktion vermieden werden. Anwendungsgrenzen natürlicher Öle und der ihnen zugrundeliegenden Fettsäuren ergeben sich aus ihrer geringen Stabilität gegen thermisch-oxidative Belastung und Hydrolyse sowie dem begrenzten Kältefließverhalten, d. h. Eigenschaften, die auch durch Additivierung mit Chemikalien wie Sulfonaten, Phenolderivaten oder Aminen nur graduell beeinflussbar sind. Züchterische Maßnahmen zur Anreicherung bestimmter Fettsäuren in den Triglyceriden von Pflanzenölen können zur Verbesserung der technischen Eigenschaften führen. Wichtiger sind jedoch chemische Umwandlungen, die sowohl an der Alkoholkomponente als auch an der Säurekomponente angreifen können.

Angriffspunkt für die oxidative Alterung von natürlichen Ölen und der ihnen zugrundeliegenden Fettsäuren sind die hierin enthaltenen Doppelbindungen, wobei speziell Doppelbindungen mit (Z)-Konformation leicht von Sauerstoff unter Bildung peroxidischer Zwischenprodukte angegriffen werden.

Zur Erhöhung der Oxidationsstabilität natürlicher Öle oder der ihnen zugrundeliegenden Fettsäuren dient z. B. der partiellen Hydrierung der mehrfach ungesättigten Säuren.

Die Addition von Ameisen- oder Essigsäure an Ölsäuremethylester und nachfolgende Hydrolyse der erhaltenen Ester ist bekannt [H.B. Knight, R.E. Koos, D. Swern, J. Am. Chem. Soc., 1953, 75, 6212; H.B. Knight, R.E. Koos, D. Swern, J. Am. Oil Chem. Soc., 1954, 31, 1]. Siedende wasserfreie Ameisensäure addiert unkatalysiert an Ölsäure mit 80% Umsatz in 24 Stunden. Werden kleine Mengen Perchlorsäure zugegeben, so wird die Reaktionszeit auf 5 Minuten verkürzt. Essigsäure addiert unkatalysiert nur in vernachlässigbarem Maße an Ölsäure; Katalyse durch Perchlorsäure führt zu 40% Umsatz in 15 Minuten, bei dreifachem Überschuß Essigsäure werden nach 70 Stunden Umsätze von 60 bis 70% erzielt [W.O. Munns, S. Kairys, D.A. Manion, J. Am. Oil Chem. Soc., 1963, 40, 22].

Ziel aller beschriebenen Verfahren ist nicht die Darstellung der Formyloxy- bzw. Acetoxyverbindungen, sondern deren Hydrolyse zur Monohydroxystearinsäure, die eine wichtige Komponente zur Herstellung von Schmierfetten und -ölen sowie Weichmachern ist.

Die Acetoxylierung zur Gewinnung der Monoester, die als Weichmacher für PVC von technischem Interesse sind, wurde unter Katalyse mit dem sauren lonentauscher Amberlyst 15 untersucht [I. T. Bleck, R.E. Beal, J. Am. 011 Chem. Soc., 1967, 44, 310]. Bei einem Massenverhältnis Methyloleat / Katalysator von zwei und einem 50-fachen molaren Überschuss von Essigsäure werden über eine Zeit von 8 Stunden Ausbeuten von 42% Methylacetoxystearat erzielt. Bei. Einsatz von Ameisensäure konnte kein katalytischer Effekt von Amberlyst 15 festgestellt werden. Die Addition von Propionund Buttersäure wurde ebenfalls untersucht; diese Säuren ergaben aber geringere Ausbeuten als Essigsäure. Es wurden keine verzweigten Carbonsäuren eingesetzt. Es wurde jedoch keine systematische Untersuchung der rheologischen Eigenschaften der erhaltenen Addukte durchgeführt. Die Regeneration des Katalysators wird in der Literatur nicht beschrieben. Ziel dieser Versuche war eine alternative Route zur Herstellung von Monohydroxystearinsäuremethylester, die durch Verseifung der Zwischenprodukte Acetoxy- und Formyloxystearinsäuremethylester erhalten werden.

Des weiteren wurden Methylacetoxyoctadecanoate synthetisiert und charakterisiert [A.P. Tulloch, Organic Megnetic Resonance, Vol. 11 (3), 1978, 109-115*].*

In keiner der aus den vorstehend aufgeführten Publikationen bekannt gewordenen Acyloxylierungen von Fettsäuren oder Fettsäurederivaten ist die Verwendung der Reaktionsprodukte als Schmierstoff und/oder Druckübertragungsmittel erwähnt bzw. bekannt geworden.

Aus der Patentschrift US 2,115,341 sind Schmiermittelzusammensetzugen bekannt, welche ein viskoses Kohlenwasserstofföl enthalten, welche ein Produkt enthalten, welches erhalten wurde durch Veresterung von zumindest einem Teil der OH-Gruppen einer Hydroxysäurenkomponente einer langkettigen Fettsäure mit einem Esterbildenden, aliphatischen Reagens, welches ein geringes Molekulargewicht aufweist, die Gruppe R-(CO)- enthält und aus der Gruppe der Säuren, Anhydride und Säurchalogenide ausgewählt ist. Im Einzelnen werden Schmierstoffzusammensetzungen in der US 2,115,341 beschrieben, die Mineralöte als Basisflüssigkeit neben bis zu 6 Gew.-% an beispielsweise mit Acetylchlorid an der Hydroxylgruppe veresterten Hydroxycarbonsäureestern enthalten. Durch die Zugabe von derartig acylierten Ricinolsäure-Glycerinester (Castoröl) soll die Schmierfähigkeit eines Kohlenwasserstofföls verbessert werden..

Mit Hilfe der beschriebenen Verfahren auf Basis von Fettsäuren, natürlichen Fettsäurederivaten und deren Gemischen können Schmierstoffe gewonnen werden, die überraschend die kritischen Eigenschaften dieser natürlichen Rohstoffe wie geringe Alterungsbeständigkeit, geringe Oxidationsbeständigkeit, niedrige Hydrolysestabilität und kritische Kälteeigenschaften verbessern, ohne indes deren Vorteile wie gute Schmierwirksamkeit oder geringe Temperaturabhängigkeit der Viskosität negativ zu beeinflussen. Durch die Flexibilität der Reaktion sind bestimmte Eigenschaften wie Viskosität, Kälteeigenschaften und Elastomerverträglichkeit in weitem Bereich einstellbar.

Aufgabe der vorliegenden Erfindung ist es daher einerseits neue Verbindungen auf Basis von acyloxylierten Fettsäuren. Fettsäurederivaten und deren Gemischen unter Einsatz ungesättigter Fettsäuren, auch aus natürlichen Quellen, bereitzustellen Überraschend wird diese Aufgabe durch die Bereitstellung von Verbindungen gemäß Anspruch 5 gelöst. Weiterhin ist die Verwendung eines Reaktionsproduktes aus acyloxylierten Fettsäuren oder Fettsäurederivaten als Schmierstoff und/oder Druckübertragungsmittel, wie in Patentanspruch 1 beschrieben, sowie ein Herstellungsverfahren nach Anspruch 6 Gegenstand der vorliegenden Erfindung. Bevorzugte Ausführungsformen sind in den Unteransprüche beschreiben.

Erfindungsgemäß wurden neuartige Schmierstoffe und Druckübertragungsmittel basierend auf Fettsäuren und/oder Fettsäurederivaten gefunden, die an mindestens einer Doppelbindung acyloxyliert sind, mit Carbonsäureresten der allgemeinen chemischen Formel I: 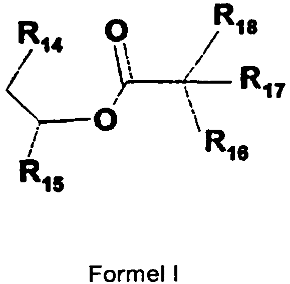
mit R₁₄ = Carbonsäurereste R-COOH mit R = Alkyl-, Alkenyl-, Dienyl oder Polyenylrest mit 2 bis 20 C-Atomen und 0 bis 4 Doppelbindungen, vorzugsweise mit 3 bis 11 C-Atomen und 0 bis 2 Doppelbindungen, z.B. n-Carboxyheptyl-, n-Carboxyundecyl-, n-Carboxy-4-heptenyl- oder deren Ester mit Monoalkoholen, Diolen oder Polyolen wie Methanol, Ethanol, Glycerin, Pentaerythrit oder Trimethylolpropan;
mit R₁₅= Alkyl-, Alkenyl-, Dienyl- oder Polyenylrest mit 2 bis 20 C-Atomen und 0 bis 4 Doppelbindungen, vorzugsweise 3 bis 14 C-Atomen und 0 bis 3 Doppelbindungen, insbesondere 3 bis 10 C-Atomen und 0 bis 2 Doppelbindungen, z.B. n-Hexyl-, n-Octyl-, 2-Octenyl- oder 2,5-Octadienyl-;
und mit R₁₆ = H oder R₁₆ =
   Alkylreste mit 1 bis 10 C-Atomen, z.B.
      Methyl-, Ethyl-, Propyl-, Isopropyl-, Butyl-, Isobutyl-, tert-Butyl-, Pentyl-, Hexyl, Octyl-, Decyl-, Cyclohexyl-;
   Arylreste mit Alkyl- und anderen Substituenten am aromatischen Ring, z.B.
      Phenyl-, Methylphenyl, Ethylphenyl-, Mesityl-, Cumyl-, p-Nitrophenylxylyl-, Hydroxyphenyl-, Naphtyl-;
   Aralkylreste, z.B.
      Benzyl-, 1 -Phenylethyl-, 2-Phenylethyl-, 2-Phenylpropyl-, Cumylmethyl-, Mesitylmethyl-;
   Alkyloxyreste mit 1 bis 10 C-Atomen und Phosphor oder Schwefel als weiteren Heteroatomen, z.B.
      Methoxy-, Ethoxy-, Propyloxy-, Butyloxy-, 2-Ethylbutyloxy-, 3-Thiabutyloxy-, 3-Phosphabutyloxy-
   Aralkyloxyreste mit Alkyl- und heteroatomhaltigen Substituenten am aromatischen Ring, z.B.
      Benzyloxy-, 1 -Phenylethyloxy-, 2-Methyl-phenylmethoxy-, p-Sulfobenzyloxy-, p-Nitrobenzyloxy-;
und mit R₁₇ = R₁₈ = R₁₆ ausgenommen R₁₆ = H,
wobei R₁₆, R₁₇ und R₁₈ gleich oder verschieden und, im Falle von R₁₆ = H, R₁₇ und R₁₈ gleich oder verschieden sein können.

Acyloxylierung im Sinne der vorliegenden Erfindung bezeichnet die Einführung eines Acyloxy-Restes der Formel -O-CO-R in eine organische Verbindung, wie beispielsweise bei Römpp, 9. Auflage, 1995, Seite 47 beschrieben, vorzugsweise durch Addition einer Carbonsäure oder eines Carbonsäurederivates an die Doppelbindung einer Fettsäure oder eines Fettsäurederivates.

Zur Herstellung von Verbindungen der allgemeinen Formel 1 können als Doppelbindungskomponenten Verbindungen der allgemeinen Formel II 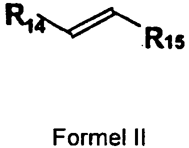 eingesetzt werden
mit R₁₄ =Carbonsäurereste R-COOH mit R = Alkyl-, Alkenyl-, Dlenyl- oder Polyenylrest mit 2 bis 20 C-Atomen und 0 bis 4 Doppelbindungen, vorzugsweise mit 3 bis 11 C-Atomen und 0 bis 2 Doppelbindungen, z.B, n-Carboxyheptyl-, n-Carboxyundecyl-, n-Carboxy-4-heptenyl- oder deren Ester mit Monoalkoholen, Diolen oder Polyolen wie Methanol, Ethanol, Glycerin, Pentaerythrit oder Trimethylolpropan:
und mit R₁₅ = Alkyl-, Alkenyl-, Dienyl- oder Polyenylrest mit 2 bis 20 C-Atomen und 0 bis 4 Doppelbindungen, vorzugsweise 3 bis 14 C-Atomen und 0 bis 3 Doppelbindungen, insbesondere 3 bis 10 C-Atomen und 0 bis 2 Doppelbindungen, z.B. n-Hexyl-, n-Octyl-, 2-Octenyl- oder 2,5-Octadienyl-.

Derartige Doppelbindungskomponenten können vertreten werden durch Ölsäure, Linolsäure, Linolensäure, Palmitoleinsäure, Eicosensäure und Erucasäure und deren Gemische, durch Otsäuremethylester, Linolsäuremethylester, Linolensäuremethylester, Palmitoteinsäuremethylester.

Als Doppelbindungskomponenten können auch Glycerintrioleat, Glycerintrilinolat, Glycerintrilinoleat, Trimethylolpropantrioleat, Trimethylolpropantrilinolat, Trimethylolpropantrilinoleat, Pentaerythrittettraoleat, Pentaerythrittetralinolat und Pentaerythrittetralinoleat und deren Gemische sowie nachwachsende Rohstoffe wie Rapsöl, Sonnenblumenöl, Palmöl, Kokosöl oder Olivenöl eingesetzt werden.

Als Doppelbindungskomponenten können auch mehrfach ungesättigte Fettsäuren und/oder Fettsäurederivate eingesetzt werden, bei denen ein Teil der Doppelbindungen zunächst hydriert wird und bei denen dann an die verbleibenden Doppelbindungen Carbonsäuren und/oder Carbonsäurehalogenide und/oder Carbonsäureanhydride addiert werden.

Zur Herstellung der erfindungsgemäßen neuen Schmierstoffe und Druckübertragungsmittel soll mindestens eine Doppelbindung der Fettsäuren und/oder Fettsäurederivate und ihrer Gemische mit. 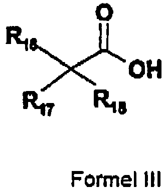
acyloxyliert werden
   mit R₁₆ = H oder R₁₆ =
   Alkylreste mit 1 bis 10 C-Atomen, z.B.
      Methyl-, Ethyl-, Propyl-, Isopropyl-, Butyl-, Isobutyl-, tert-Butyl-, Pentyl-, Hexyl, Octyl-, Decyl-, Cyclohexyl-;
   Alkenylreste mit 1 bis 10 C-Atomen, z.B.
      Ethenyl-, n-Prapenyl-, Isopropenyl-, Hexenyl-, 3-Methylpentenyl, 3-Ethylbuteny-;
   Arylreste mit Alkyl- und anderen Substituenten am aromatischen Ring, z.B.
      Phenyl-, Methylphenyl, Ethylphenyl-, Mesityl-, Cumyl-, p-Nitrophenyl-Xylyl-, Hydroxyphenyl-, Naphtyl-;
   Aralkylreste, z.B.
      Benzyl-, 1 -Phenylethyl-, 2-Phenylethyl-, 2-Phenylpropyl-, Cumylmethyl-, Mesitylmethyl-;
   Alkyloxyreste mit 1-10 C-Atomen und Phosphor oder Schwefel als weiteren Heteroatomen, z.B.
      Methoxy-, Ethoxy-, Propyloxy-, Butyloxy-, 2-Ethylbutyloxy-, 3-Thiabutyloxy-, 3-Phosphabutyloxy-
   Aralkyloxyreste mit Alkyl- und heteroatomhaltigen Substituenten am aromatischen Ring, z.B.
      Benzyloxy-, 1 -Phenymethyloxy-, 2-Methyl-phenylmethoxy-, p-Sulfobenzyloxy-, p-Nitrobenzyloxy-;
und mit R₁₇ = R₁₈ = R₁₆ ausgenommen R₁₆ = H,
wobei R₁₆, R₁₇, und R₁₈ gleich oder verschieden und, im Falle von R₁₆ = H, R₁₇ und R₁₈ gleich oder verschieden sein können.

Besonders bevorzugt werden Neocarbonsäuren der allgemeinen Formel V: 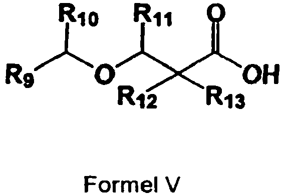 addiert, in der R₉, R₁₀, R₁₂ und/oder R₁₃ gleich oder verschieden sind und Wasserstoff, geradkettige oder verzweigte Alkyl-, Alkenyl, oder Alkinylreste mit bis zu 18 Kohlenstoffatomen, Cycloalkyl- oder Cycloalkenylreste mit 5 bis 8 Kohlenstoffatomen, Aryl-Aralkyl- oder Alkenylarylreste mit 6 bis 16 Kohlenstoffatomen oder heterocyclische Reste bedeuten, wobei jeweils die Reste R₉ und R₁₀ und/oder R₁₂ und R₁₃ zusammen mit dem Kohlenstoffatom, an das sie gebunden sind, ein Cycloalkan, Cycloalken oder einen Heterocyclus mit 5 bis 7 Ringgliedern bilden können, R₉, R₁₀, R₁₂ und R₁₃ überdies Substituenten, insbesondere solche, die unter den Reaktionsbedingungen inert sind, tragen können und R₁₁ für Wasserstoff oder einen geradkettigen oder verzweigten Alkylrest steht.

Bevorzugt sind Carbonsäuren, in denen R₉, R₁₀, R₁₂ und R₁₃ gleich oder verschieden sind und Wasserstoff, geradkettige oder verzweigte Alkylreste mit 1 bis 12, insbesondere 1 bis 6 Kohlenstoffatomen, Alkenyl- oder Alkinylreste mit 2 bis 12, insbesondere 2 bis 6 Kohlenstoffatomen bedeuten. Bevorzugt sind ferner Verbindungen, in denen R₉, R₁₀, R₁₂ und R₁₃ für Cycloalkyl- oder Cycloalkenylreste mit 5 oder 6 Kohlenstoffatomen, für Aryl-, Alkylaryl-, Aralkyl- oder Alkenylarylreste mit 6 bis 12 Kohlenstoffatomen und für heterocyclische Reste, die ein oder mehrere Stickstoff- und/oder Sauerstoff- und/oder Schwefelatome enthalten, stehen. Weiterhin werden Neocarbonsäuren bevorzugt, in denen R₁₁ Wasserstoff oder ein geradkettiger oder verzweigter Alkylrest mit 1 bis 12, insbesondere 1 bis 8, vorzugsweise 1 bis 4, Kohlenatoffatomen ist. Besonders bevorzugt steht R¹¹ für Wasserstoff.

Beispiele für Alkyl-, Alkenyl- oder Alkinylreste sind der Methyl-, Ethyl-, n-Propyl-, Isopropyl-, Propenyl-, Isopropenyl-, n-Butyl-, Isobutyl-, n-Butenyl-, i-Butenyl-, n-Butinyl-, Pentyl-, Pentenyl-, Pentinyl-, Hexyl-, Hexenyl-, Heptyl-, Heptenyl-, Octyl-, Octenyl-, Nonyl-, Nonenyl-, Decyl-, Decenyl-, Dodecyl- oder Dodecenylreste.

Cycloalkylreste werden beispielhaft durch den Cyclopentyl-, Cyclohexyl-, Cycloheptyl-, Cyclopentenyl- oder Cyclohexenylrest vertreten.

Als aromatische Reste kommen z. B. der Phenyl-, Benzyl-, 2-Phenylethyl-, 2-Phenylpropyl-, 3-Phenylpropyl-, 2-Phenylbutyl-, 3-Phenylbutyl oder 4-Phenylbutylrest in Betracht.

Beispiele für heterocyclische Reste sind der Furan-, Dihydrofuran-, Tetrahydrofuran-, Thiophen-, Dihydrothiophen-, Pyridin- und Thiopyranrest.

Die Alkyl-, Cycloalkyl- aromatischen und heterocyclischen Reste können substituiert sein, insbesondere durch Reste, die unter Reaktionsbedingungen inert sind, wie Halogen-, Alkoxy-, Carboxy- oder Carboxylatgruppen. Es ist aber in Einzelfällen auch nicht ausgeschlossen, dass bewusst Substituenten ausgewählt werden, die im Verlauf der Reaktion verändert werden, z. B. Formylgruppen, die in Formoxyreste überführt werden.

In dieser Weise können erfindungsgemäß beispielsweise die folgenden in alpha-Position zur Carboxylgruppe verzweigten Carbonsäuren eingesetzt werden wie:

### 3-Propyloxy-2,2-dimethylpropionsäure

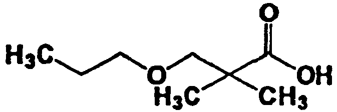

Die spektroskopischen Daten dieser Verbindung wurden wie nachfolgend angegeben bestimmt:
- **¹H-NMR 2H**: 0,89ppm tr 3H, 1,19ppm 2 6H, 3,42ppm s 2H, 3,40ppm s 2H, 1,56ppm m
- **¹³C-NMR**: 10,66ppm CH₃, 23,06ppm CH₂, 77,54 und 73,69ppm O-CH₂, 43,83ppm quarternäres C, 22,46ppm 2 CH₃, 183,01 ppm C=O
- **IR**: 1706cm-¹ C=O, 2976, 2935, 2879cm⁻¹ v_{s.as} CH₂, CH₃, breite Schulter bei 2700-2500 cm⁻¹
- **MS**: m/z: 175 (M⁺.), 131, 115, 101, 102, 87, 83

### 3-Butyloxy-2,2-dimethylpropionsäure

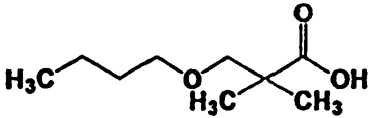

Hierzu wurden folgende spektroskopische Daten bestimmt:
- **¹H-NMR**: 0,90ppm tr 3H, 1,34ppm m 2H, 1,53ppm q 2H, 3,45ppm tr 2H, 3,42ppm s 2H, 1,19ppm s 6H
- **¹³C-NMR**: 14,01ppm CH₃, 19,69ppm CH₂, 31,95ppm CH₂, 77,65 und 71,80ppm O-CH₂, 43,87ppm quarternäres C, 22,44ppm 2 CH₃, 183,72ppm C=O
- **IR**: 1706cm⁻¹ C=O, 2971, 2935, 2879cm⁻¹ v_{s.as} CH₂, CH₃ breite Schulter bei 2700-2500cm⁻¹
- **MS**: m/z: 189 (M⁺.), 131, 114, 102, 101, 87, 73

### 3-Isobutoxy-2,2-dimethylpropionsäure

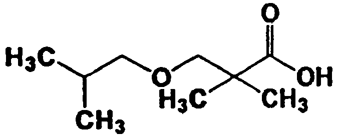

Folgende spektroskopische Daten wurden zu dieser Verbindung gemessen:
- **¹H-NMR**: 0,88ppm s 3H, 0,89ppm s 3H, 1,84ppm m 1H, 3,20ppm d 2H, 3,41ppm s 2H, 1,20ppm s 6H
- **¹³C-NMR**: 19,36ppm 2 CH₃, 28,70ppm CH, 78,83 und 77,75ppm O-CH₂, 22,44ppm 2 CH₃, 43,87ppm quarternäres C, 183,04ppm C=O
- **IR**: 1706cm⁻¹ C=O, 2976, 2935, 2868cm⁻¹ v_{s.as} CH₂, CH₃, breite Schulter bei 2700-2500cm⁻¹
- **MS**: m/z: 189 (M⁺.), 131, 115, 102, 101, 87, 73

### 3-(2-Ethylbutyloxy)-2,2-dimethylpropionsäure

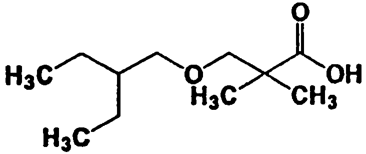

Für diese Verbindung wurden folgende spektroskopische Daten gemessen:
- **¹H-NMR**: 0,93ppm tr 3H, 0,86ppm tr 3H, 1,59ppm m 4H, 2,22ppm m 1H, 3,33ppm d 2H, 3,40ppm s 2H, 1,19ppm s 6H
- **¹³C-NMR**: 11,87 und 11,26ppm CH₃, 23,86 und 25,16ppm CH₂, 49,34ppm CH, 77,92 und 74,36ppm O-CH₂, 22,46ppm 2 CH₃, 43,90ppm quarternäres C, 183,16ppm C=O
- **IR**: 1706cm⁻¹ C=O, 2966, 2935, 2879cm⁻¹ v_{s.as} CH₂, CH₃ breite Schulter bei 2700-2500cm⁻¹
- **MS**: m/z: 217 (M⁺.), 185, 145, 133, 131, 115,102, 101, 85, 73

### 3-Benzyloxy-2,2-dimethylpropionsäure

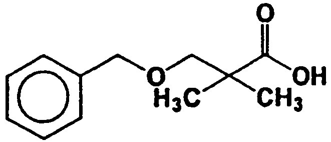

Die Verbindung besitzt folgende spektroskopische Daten:
- **¹H-NMR**: 7,20ppm m 5H, 4,37ppm s 2H, 3,35ppm s 2H, 1,08ppm s 6H
- **¹³C-NMR**: 125-130ppm aromatische C, 77,13 und 73,28ppm 0-CH₂, 21,87ppm 2 CH₃, 43,80ppm quarternäres C, 182,89ppm C=O
- **IR**: 1701cm⁻¹ C=O, 3094, 3063, 3033cm⁻¹ vₐᵣ CH, 2984, 2909, 2878cm-1 v_{s.as} CH₂, CH₃, breite Schulter bei 2700-2500cm⁻¹
- **MS**: m/z: 222 (M⁺.), 191, 116, 107, 101, 91, 65

### 3-(2-Methylbenzyloxy)-2,2-dimethylpropionsäure

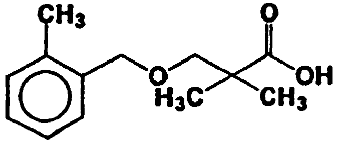

Die Verbindung ergab folgende spektroskopische Daten:
- **¹H-NMR**: 7,0 - 7,3ppm m 4H, 2,28ppm s 3H, 3,48ppm s 2H, 3,44ppm s 2H, 1,21ppm s 6H
- **¹³C-NMR**: 125 - 130ppm aromatische C, 77,31 und 72,28ppm O-CH₂, 43,78ppm quarternäres C, 22,46ppm CH₃-ar, 18,82 und 15,32ppm CH₃, 182,79ppm C=O
- **IR**: 1703cm⁻¹ C=O, 3073, 3027cm⁻¹ vₐᵣ CH, 2976, 2935, 2868cm⁻¹ v_{s.as} CH₂, CH₃, breite Schulter bei 2700-2500cm⁻¹
- **MS**: m/z: 237 (M⁺.),209, 193, 145, 131, 121, 115, 105

Die vorstehend genannten Carbonsäuren werden erhalten über die katalytische Gasphasenisomerisierung entsprechend substituierter 1,3-Dioxane zu Aldehyden und deren anschließende Oxidation zu den Carbonsäuren. Die Umsetzung von 1,3-Dioxanen zu Aldehyden ist in der EP-A-0 199 210 beschrieben.

Als Isomerisierungskatalysatoren kommen saure Feststoffe zum Einsatz. Dies sind Metalloxide wie Al₂O₃, TiO₂, SiO₂, ZrO₂, Nb₂O₅ oder Phosphate wie Borphosphat, Aluminiumphosphat oder Eisenphosphat oder sulfatiertes Zirkon- oder Titandioxid, aber auch Ionenaustauscherharze wie Amberlyst, Nafion und Nafion/Silica Composite.

Als Katalysatoren für die Oxidation der durch die Isomerisierung der Dioxane erhaltenen Aldehyde werden zweckmäßig Metalle der I. und VIII. Nebengruppe des Periodensystems der Elemente in metallischer Form oder in Form ihrer Oxide eingesetzt. Diese Katalysatoren können rein oder geträgert eingesetzt werden. Bevorzugt ist die Verwendung von Silber oder Silber(I)oxid, wobei das Oxid in reiner Form und das Silbermetall auf Trägern zum Einsatz kommt.

Zur Herstellung der erfindungsgemäßen neuen Schmierstoffe und Druckübertragungsmittel können die Doppelbindungen der natürlichen Fettsäuren, natürlicher Fettsäurederivate und ihrer Gemische statt mit Carbonsäuren mit den entsprechenden Carbonsäurenanhydriden und/oder Carbonsäurehalogeniden acyloxyliert werden. Die eingesetzten Fettsäuren bzw. Fettsäurederivate können von natürlichem oder synthetischem Ursprung sein. Auch kann bei bestimmten Carbonsäurekomponenten bei der Acyloxylierung der Einsatz weiterer Lösungsmittel überflüssig werden, statt dessen kann die Carbonsäurekomponente selbst als Lösungsmittel in einem molaren Überschuss im Bereich von 1,2 bis 150 Mol-% eingesetzt werden.

Die Reaktionen werden in Gegenwart von homogenen oder heterogenen Katalysatoren durchgeführt. Es konnte gezeigt werden, dass durch Verwendung homogener oder heterogener acider Katalysatoren gute Umsätze und hohe Selektivitäten in der Additionsreaktion erreicht werden können.

Von besonderem Vorteil sind erfindungsgemäß heterogene Katalysatoren wie Zeolithe, feste Phosphorsäure, Phosphate des Aluminiums, Bors, Eisens, Strontiums, Cers und Zirconiums, Oxide wie Al₂O₃, SiO₂, B₂O₃, Fe₂O₃, ZrO₂, SnO₂ und GeO₂ und organische Ionentauscher wie Amberlyst oder Nafion, die leicht von den gebildeten Produkten abzutrennen sind. Die Verwendung von Zeolithen als heterogene Katalysatoren ist bekannt [H. Kessler, Comprehensive Supramolecular Chemistry Volume 7: Solid-state Supramolecular Chemistry: Two- and Three-dimensional Inorganic Network 1996, pp. 425-464; L.B. McCusker, Comprehensive Supramolecular Chemistry Volume 7: Solid-state Supramolecular Chemistry: Two- and Three-dimensional Inorganic Network 1996, pp. 393-424], ebenso wie die Verwendung von Aluminiumphosphaten [E.M. Flanigen, B.M. Lok, R.L. Patton, S.T. Wilson in Y. Murakaml, A. Ijma and J.W.Ward (Eds.), New Developments in Zeolite Science and Technology, Proc. 7th Intl. Zeolite Conf., Tokyo, 1986, Kodansha Ltd., Tokyo and Elsevier, Amsterdam, pp. 101-112]. Insbesondere Nafion-Sifica-Composite-Materialien liefern gute Ergebnisse. Hierbei ist es von Vorteil, den Katalysator vor Einsatz in der Reaktion mehrere Stunden bei erhöhter Temperatur im Vakuum zu trocknen. Die genannten Heterogenkatalysatoren sind leicht regenerierbar und können daher wiederholt in das Verfahren eingesetzt werden, was technisch besonders vorteilhaft ist. Während sich Ameisensäure auch ohne Katalysator an die Doppelbindung natürlicher Fettsäuren und Fettsäurederivate addiert, wird bei Einsatz höherer Carbonsäuren in Abwesenheit von Katalysatoren kein oder nur sehr geringer Umsatz erzielt, die Reaktion mit Carbonsäuren kann in einzelnen Fällen auch autokatalytisch verlaufen.

Als homogene acide Katalysatoren können unter anderem H₂SO₄, Methylsulfonsäure, H₃PO₄ und deren Derivate, H₃BO₃, HNO₃, HCl, HF, HF-BF₃, AlCl₃. FeCl₃, SnCl₂, AlBr₃ und FeBr₃ zum Einsatz kommen.

Erfindungsgemäß konnten neuartige Ester natürlicher Fettsäuren und Fettsäurederivate entsprechend nach folgender Gleichung 1 hergestellt werden:

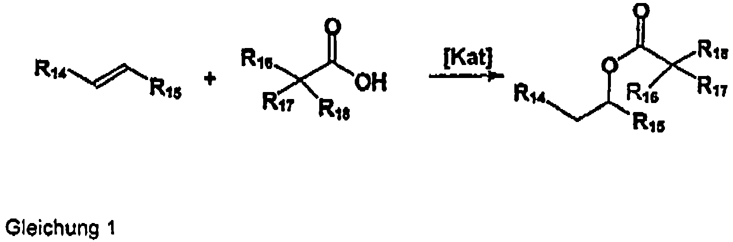

Die Analytik wurde über HPLC (Hochdruckflüssigchromatographie) und GC (Gaschromatographie) durchgeführt. Zusätzliche Hinweise auf die Identität der hergestellten Produkte wurden durch Massenspektrometrie (MS) erhalten. Die gebildeten Additionsprodukte können durch Destillation oder Säulenchromatographie von ihren Ausgangsprodukten getrennt und In einer Reinheit im Bereich von 70 bis 99 % isoliert und danach NMR- und IR-spektroskapisch charakterisiert werden.

Beispielsweise besitzt der Ester mit der folgenden chemischen Formel (Verbindung III) folgende charakteristische Merkmale: 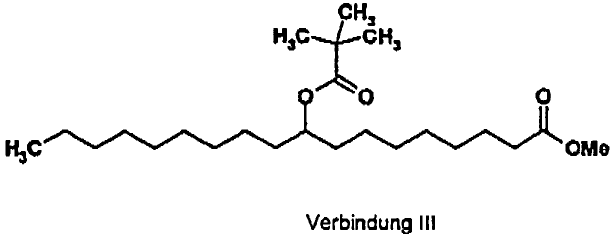
- **¹H-NMR**: 0,8 ppm tr 3H (Methylgruppe Kettenende), 1,13ppm s 9H (tert.-Butylgruppe), 1,19 - 1,6ppm mehrere breite Peaks (Methylengruppen der Kette), 2,12ppm tr 2H (CH₂-Gruppe in Nachbarschaft zur Methylestergruppe), 3,59ppm s 3H (O-CH₃-Gruppe) und 4,78ppm q 1H (estersubstitulerte CH-Gruppe)
- **¹³C-NMR**: 14,12ppm (CH₃-Gruppe Kettenende), 27,25ppm (tert.-Butylgruppe), 7 Peaks von 22,66 - 34,14ppm (Methylengruppen der Kette), 38,84ppm [(CH₃)₃C], 51,44ppm (O-CH₃-Gruppe), 73,81 ppm (estersubstituierte CH-Gruppe), 174,35ppm (-CH₂CO₂CH₃), 178,27ppm ((CH₃)₃C-CO₂-CH)
- **IR**: 1164 cm⁻¹ vₐₛ C-O-C, 1283 cm⁻¹ vₐₛ C-O-C, 1435 cm⁻¹ δs CH₂/-CH₂-CO-C, 1462 cm⁻¹ δas CH₃, 1480 cm⁻¹ δs CH₂, 1742, 1726 cm⁻¹ C=O, 2950, 2926, 2856cm⁻¹ vₛ,ₐₛ CH₂, CH₃
- **MS**: m/z: 399 (M⁺.), 313, 297, 265, 247, 220, 111, 97, 95, 83, 69, 57, 55

| | | |
|---|---|---|
| Siedepunkt: 170 - 174°C/1·10⁻¹ mbar | | |
| Elementaranalyse: | C_{gemessen}: 72,4 % | C_{berechnet}: 72,3 % |
| | H_{gemessen}: 12,0 % | H_{berechnet}: 11,6 % |

Es ist erfindungsgemäß für die Esterbildung vorteilhaft, die Carbonsäure und/oder das Carbonsäureanhydrid und/oder die Carbonsäurehalogenide im Überschuß und als Lösungsmittel einzusetzen, wodurch die Verwendung zusätzlicher Solventien vermieden wird.

Die Addition der Carbonsäure erfolgt an die Doppelbindungen der Oleochemikalien Komponente. Durch den Carbeniumion-Mechanismus kann in geringem Maße Doppelbindungsisomerisierung auftreten. Als Nebenprodukte der Reaktion können Polymere entstehen sowie in geringem Ausmaß Umesterungsprodukte durch Spaltung der Fettsäureester und Veresterung der im Überschuss vorliegenden Carbonsäurekomponente.

Die Heterogenkatalysatoren können nach erfolgter Reaktion leicht abfiltriert, gewaschen und regeneriert werden, wobei die Anfangsaktivität wiederhergestellt werden kann.

Nicht abreagierte Carbonsäuren bzw. deren Derivate können ebenso wie die ÖlKomponente destillativ oder chromatographisch von dem gebildeten Produkt abgetrennt werden. Ebenso können die gebildeten Additionsprodukte durch Destillation oder Säulenchromatographie in einer Reinheit von 70 bis 99 % isoliert und NMR- und IRspektroskopisch charakterisiert werden.

Die Addition der Carbonsäure bzw. derer Derivate an die natürlichen Fettsäuren und natürlichen Fettsäurederivate erfolgt in der Flüssigphase und bei Temperaturen im Bereich von 0 bis 400°C, bevorzugt von 25 bis 250°C, insbesondere von 70 bis 180°C. Die Reaktion wird bei Drücken im Bereich von 1000 bis 10 000 hPa, bevorzugt von 1000 bis 5000 hPa, insbesondere von 1000 bis 2000 hPa durchgeführt. Es werden Belastungen von 0,001 mmol bis 50 mol Edukt pro Gramm Katalysator verwendet, bevorzugt von 0,01 mmol bis 30mol, insbesondere von 0,1 bis 10 mol.

Die Reaktionszeit liegt im Bereich zwischen einer Minute und 12 Tagen, bevorzugt im Bereich von 10 Minuten bis 5 Tagen, insbesondere von einer Stunde bis 48 Stunden. Der molare Überschuss der Säurekomponente im Verhältnis zur Ölkomponente beträgt 0,3 bis 300, bevorzugt 1,2 bis 150, insbesondere 10 bis 80.

Höhere Verzweigung der addierten Carbonsäure bzw. Carbonsäurederivate vermindert normalerweise ihre Reaktivität. Gleichzeitig wird jedoch die Selektivität der Reaktion deutlich gesteigert, da durch die geringere Säurestärke Nebenreaktionen wie Polymerisation und Umesterung weniger stark auftreten. Beim Scale-up der Reaktion können die guten Selektivitäten und Umsätze beibehalten werden, jedoch sind manchmal längere Reaktionszeiten erforderlich.

Die Reaktion kann in diskontinuierlichem oder kontinuierlichem Verfahren durchgeführt werden. So können im diskontinuierlichen drucklosen Verfahren als Reaktoren Rührkessel zum Einsatz kommen; bei Arbeiten unter Druck können Edelstahlautoklaven verwendet werden. In halbkontinuierlicher Fahrweise werden Rührkesselkaskaden eingesetzt. In kontinuierlicher Reaktionsführung werden bevorzugt Rohrreaktoren mit Katalysatorfestbett verwendet. Weiterhin können Schlaufen- und Rieselbettreaktoren eingesetzt werden.

Zur Durchführung der Reaktion wird zu den als Edukt verwendeten nativen Fettsäuren bzw. nativen Fettsäurederivaten in Gegenwart des Katalysators bei Umgebungstemperatur unter Rühren die Säurekomponente zugegeben und der Kolben in ein Heizbad der gewünschten Reaktionstemperatur gegeben. Die Reaktion erfolgt unter Rühren und Kühlung mittels eines Rückflusskühlers. Zur Analytik wird in verschiedenen Zeitabständen eine definierte Probenmenge entnommen und mittels HPLC oder GC untersucht. Nach Abschluss der Reaktion wird der Katalysator abfiltriert, mit einem organischen Lösungsmittel gespült und mit Salz- oder Salpetersäure regeneriert. Die Regenerierung kann auch in Sauerstoffatmosphäre durch Abbrennen von Kohlenstoffablagerungen erfolgen (Zeolithe). Das Reaktionsgemisch wird durch HV-Destillation oder Säulenchromatographie fraktioniert und verbliebene Öl- und Säurekomponente in die Reaktion zurückgeführt.

Die eingesetzte Ölkomponente kann durch vorherige partielle Hydrierung modifiziert werden.

Hierzu können die natürlichen Fettsäuren oder natürlichen Fettsäurederivate unter erhöhtem Druck mit Wasserstoff an verschiedenen heterogenen Katalysatoren, z. B. Nickel, Platin oder Palladium auf Trägermaterialien, bei Temperaturen um 150°C in der Flüssigphase umgesetzt werden. An die verbleibenden Doppelbindungen können nach obiger Beschreibung Carbonsäuren katalytisch addiert werden.

Die erfindungsgemäßen neuen Schmierstoffe und Druckübertragungsmittel zeigen eine hohe Oxidations- und Hydrolysebeständigkeit, verbesserte Viskositätseigenschaften mit breitem Viskositätsbereich, geringerem Dampfdruck und optimierter Schmierfilmbildung sowie verringerte Reibmomente z.B. im Spindellager. Sie sind biologisch gut abbaubar. Die erfindungsgemäßen Substanzen bzw. Substanzgemische können auch als Funktionsflüssigkeiten und Schmierstoffadditive Verwendung finden.

Die Erfindung wird im folgenden an Hand von Ausführungsbeispielen noch näher erläutert.

### Beispiele:

### Katalysator A:

Katalysator A = ^{®}Nafion SAC 13, d.h. Nafion-Silica-Composite-Material mit einem NafionGehalt von 13 %. Nafion ist eingetragenes Markenzeichen der Firma DuPont. Es handelt sich um ein perfluoriertes Polymer, welches durch Sulfonierung hochacide Säurefunktionen erhält. Durch Co-Präcipitation von Nafion mit Silicagel werden Materialien mit wechselndem Nafiongehalt mit einer Oberfläche um 300 bis 400 m²/g erhalten, die als Nafion-Composites bezeichnet werden.

### Katalysator B:

Katalysator B = ^{®}Amberlyst 15 der Firma Fluka mit einer Oberfläche von 45m²/g und einer Porosität von 32 %. Amberlyst ist ein Warenzeichen der Firma Rohm und Haas Company. Macroreticulare lonentauscher werden durch Co-Polymerisation von Styrol mit 3 bis 5% Divinylbenzol zur Vernetzung hergestellt. Die Säurefunktion wird durch SO₃H-Gruppen gebildet.

### Beispiele 1 und 2 (Ameisensäure ohne Katalysator): (nicht erfindungsgemäß)

190g Ölsäuremethylester wurden unter Rühren mit einem Magnetrührstab mit 600g Ameisensäure bei einer Temperatur von 120°C umgesetzt. Es wurde 18 h unter Rockfluss gerührt. Nach der Reaktion wurde eine Probe entnommen und mit einer 60m SE-54-Säule der Firma Chrompack gaschromatographisch untersucht.

Die Reaktion verlief entsprechend der folgenden Gleichung (3): 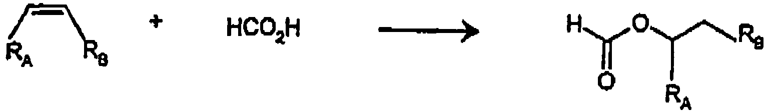 R_{A}: H₃C-(CH₂)₀-CH₂- R_{B}: -(CH₂)₇CO₂CH₃

| Beispiel | Reaktionszeit [h] | Molverhältnis; * | U [%] | S [%] | A [%] |
|---|---|---|---|---|---|
| 1 | 18 | 20 | 72,5 | 96,5 | 70,0 |
| 2 | 18 | 26 | 85,5 | 91,1 | 77,9 |

| | | | | | |
|---|---|---|---|---|---|
| * ausgedrückt als Ameisensäure/Olsäuremethylester | | | | | |

Das Reaktionsgemisch wurde im Hochvakuum fraktioniert. Hierbei trat Ameisensäure bei 54°C/150 Torr über, Ölsäuremethylester und übrige Eduktkomponenten bei 133°C/1-10⁻¹ mbar und 9(10)-Formyloxystearinsäuremethylester bei 160 - 174°C/1-10⁻¹ mbar. Es wurden 105g Produktmischung mit einem Gehalt von 90% 9(10)-Formyloxystearinsäuremethylester erhalten.

Charakterisierung der Verbindung I = 9-(10)-Formyloxystearinsäuremethylester:
- ¹H-NMR: 0,83ppm tr 3H (Methylgruppe Kettenende), 1,19 - 1,6ppm mehrere breite Peaks (Methylengruppen der Kette), 2,25ppm tr 2H (CH₂-Gruppe in Nachbarschaft zur Methylestergruppe), 3,61 ppm s 3H (O-CH₃-Gruppe), 4,78ppm q 1H (estersubstituierte CH-Gruppe), 8.04ppm s 1H (Formyloxygruppe)
- ¹³C-NMR: 14,13ppm (CH₃-Gruppe Kettenende), 7 Peaks von 22.70 - 34,09ppm (Methylengruppen der Kette), 51,44ppm (O-CH₃-Gruppe), 74.53ppm (estersubstituierte CH-Gruppe), 161,14ppm (HCO₂CH), 174,31 ppm (-CH₂-CO₂-CH₃)
- IR: 1983 cm⁻¹ vₒ₃ C-O-C (starker Peak für Formiate), 1377 cm⁻¹ δs CH₃, 1437 cm⁻¹ δs CH₂/-CH₂-CO-C, 1465 cm⁻¹ δas CH₃, 1377 cm⁻¹ δs CH₂, 1742, 1725cm⁻¹ C=O. 2928, 2856 cm⁻¹ v_{s.as} CH₂, CH₃
- MS: m/z: 343 (M⁺.) 313, 297, 265, 264, 246, 235, 222, 208, 111, 109, 81, 67, 55

| | | |
|---|---|---|
| Siedepunkt: 160 - 174°C/1.10⁻¹ mbar | | |
| Elementaranalyse: | C_{gemesson}: 70,4 % | C_{berechnot}: 70.13 % |
| | H_{gemesson}: 11,38 % | H_{berechnot}: 11,18 % |

### Beispiel 3 (Ameisensäure mit Amberlyst als Katalysator): (nicht erfindungsgemäß)

| | |
|---|---|
| Ölsäuremethylester | 2,5 g |
| Ameisensäure: | 19.55g |
| Katalysator: | 2.5g |
| Temperatur: | 120°C |

| | | | | | |
|---|---|---|---|---|---|
| Beispiel | Katalysator | Reaktionszeit [h] | U [%] | S [%] | A [%] |
| 3 | Amberlyst 15 | 8 | 99,9 | 80,8 | 80.8 |

### Beispiel 4 (Ameisensäure mit Nafion/Silica Composite SAC 13 als Katalysator): (nicht erfindungsgemäß)

| Beispiel | Katalysator | Reaktionszeit [h] | U [%] | S [%] | A [%] |
|---|---|---|---|---|---|
| 4 | Nafion SAC 13 | 8 | 83,0 | 77,3 | 64,1 |

### Beispiel 5 (Essigsäure mit Schwefelsäure als Katalysator): (nicht erfindungsgemäß)

| | | | | | |
|---|---|---|---|---|---|
| Ölsäuremethylester: | | | | 2,5g | |
| Essigsäure: | | | | 25,5g | |
| Katalysator: | | | | 2,5g | |
| Temperatur: | | | | 120°C | |

| Beispiel | Katalysator | Reaktionszeit [h] | U [%] | S [%] | A [%] |
|---|---|---|---|---|---|
| 5 | H₂SO₄ | 20 | 90,1 | 40,5 | 36,5 |

### Beispiel 6 (Essigsäure mit Phosphorsäure auf Silica als Katalysator): (nicht erfindungsgemäß)

| Beispiel | Katalysator | Reaktionszeit [h] | U [%] | S [%] | A [%] |
|---|---|---|---|---|---|
| 8 | H₃PO₄ auf SiO₂ | 24 | 80,4 | 51,4 | 41,4 |

### Beispiel 7 (Essigsäure mit Nafion/silica Composite SAC 13 als Katalysator): (nicht erfindungsgemäß)

| Beispiel | Katalysator | Reaktionszeit [h] | u [%] | S [%] | A [%] |
|---|---|---|---|---|---|
| 7 | Nafion SAC 13 | 32 | 55,2 | 4 | 51,8 |

### Analytik des Acetoxystearinsäuremethylesters

Charakterisierung der Verbindung II: 9(10)-Acetoxystearinsäuremethylester
- **¹H-NMR**: 0,81ppm tr 3H (Methylgruppe Kettenende), 1,19 bis 1,6ppm mehrere breite Peaks (Methylengruppen der Kette), 1,97ppm s 3H (CH₃-Gruppe Acetoxygruppe). 2,27ppm tr 2H (CH₂-Gruppe in Nachbarschaft zur Methylestergruppe), 3,60ppm s 3H (O-CH₃-Gruppe), 4,78ppm q 1H (estersubstituierte CH-Gruppe),
- **¹³C-NMR**: 14,15ppm (CH₃-Gruppe Kettenende), 21,34ppm (CH₃-Gruppe Acetoxygruppe), Peaks von 22,73 bis 34,17ppm (Methylengruppen der Kette), 74,53ppm (estersubstituierte CH-Gruppe), 174,13ppm (CH₂CO₂CH), 179,97ppm (CH₃C-CO₂-CH)
- **IR**: 1164 cm⁻¹ vₒ₃ C-O-C, 1283 cm⁻¹ vₒ₃ C-O-C, 1361 cm⁻¹ δs CH₃/CH₃-CO₂-, 1435 cm⁻¹ δs CH₂/-CH₂-CO-C, 1462 cm⁻¹ δas CH₃,1481 cm⁻¹ δs CH₂, 1745, 1726 cm⁻¹ C=O, 2926, 2856cm⁻¹ v_{s.o5} CH₂, CH₃
- **MS**: m/z: 356 (M⁺.), 355, 313, 295, 281, 263, 245, 187, 155, 109, 95, 81, 67, 55
- **Siedepunkt:**: 170 - 182°C/5-10⁻² mbar

### Beispiel 8:

30g Nafion SAC 13 wurden über eine Zeitdauer von vier Stunden unter Hochvakuum bei einer Temperatur von 100°C getrocknet, dann in einen 2 I-Einhalskolben gegeben und mit 100g eines Fettsäuremethylestergemisches mit einem Gehalt von 66 % Ölsäuremethylester versetzt. Unter Rühren mit einem Magnetrührstab wurden 570g Pivalinsäure zugegeben, dann wurde der Kolben in ein auf 120°C temperiertes Ölbad gegeben. Über eine Zeitdauer von 15 h wurde die Mischung unter Kühlung durch einen Rückflusskühler gerührt. Nach Abschluss der Reaktion wurde eine Probe entnommen und unter Verwendung einer 60m SE-54-Säule der Firma Chrompack gaschromatographisch untersucht. Es wurde ein Umsatz an Ölsäuremethylester von 45 % bei einer Selektivität zum Additionsprodukt von 96 % festgestellt.

Die Reaktion verläuft entsprechend Gleichung (4):

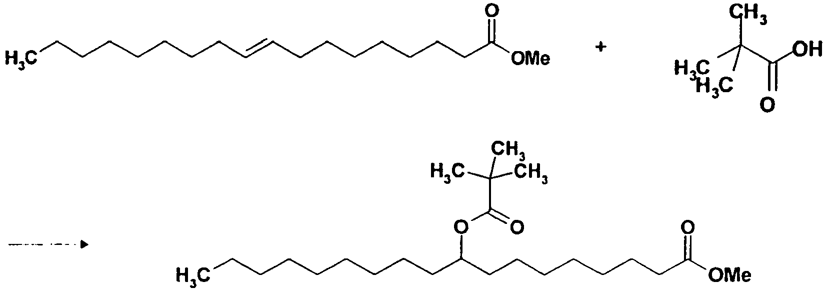

Der Katalysator wurde dann abfiltriert und durch Waschen mit Aceton von Resten des Reaktionsgemisches befreit. Das Aceton wurde anschließend bei Normaldruck abdestilliert und der verbleibende Rest mit dem Reaktionsgemisch vereinigt. Sollte der Katalysator nach einer solchen Reaktion desaktiviert sein, wird er durch Behandlung mit Salzsäure regeneriert.

Das Reaktionsgemisch wurde dann im Hochvakuum fraktioniert. Hierbei trat Pivalinsäure bei 82°C/25 Torr über, Ölsäuremethylester und übrige Eduktkomponenten bei 133°C/1·10⁻¹ mbar und 9-(10)[2,2-Dimethylpropionyloxy]-stearinsäuremethylester bei 170 bis 174°C/1·10⁻¹ mbar. Es werden 30g Produktmischung mit einem Gehalt von 70 % 9-(10)-Dimethyl-propionyloxystearinsäuremethylester erhalten.

Charakterisierung der Verbindung III: 9-(10)-[2,2'-Dimethylpropionyloxy]stearinsäure-methylester:
- **¹H-NMR**: 0,8 ppm tr 3H (Methylgruppe Kettenende), 1,13ppm s 9H (tert.-Butylgruppe), 1,19 - 1,6ppm mehrere breite Peaks (Methylengruppen der Kette), 2,12ppm tr 2H (CH₂-Gruppe in Nachbarschaft zur Methylestergruppe), 3,59ppm s 3H (O-CH₃-Gruppe), 4,78ppm q 1H (estersubstituierte CH-Gruppe)
- **¹³C-NMR**: 14,12ppm (CH₃-Gruppe Kettenende), 27,25ppm (tert.-Butylgruppe), 7 Peaks von 22,66 bis 34,14ppm (Methylengruppen der Kette), 38,84ppm {(CH₃)₃C}, 51,44ppm (O-CH₃-Gruppe), 73,81 ppm (estersubstituerte CH-Gruppe), 174,35ppm (-CH₂CO₂CH₃), 1 78,27ppm {(CH₃)₃C-CO₂-CH}
- **IR**: 1164 cm⁻¹vₐₛ C-O-C, 1283 cm⁻¹ vₐₛ C-O-C, 1435 cm⁻¹ δs CH₂/-CH₂-CO-C, 1462 cm⁻¹ δas CH₃, 1480 cm⁻¹ δs CH₂, 1742, 1726 cm⁻¹ C=O, 2950, 2926, 2856 cm⁻¹v_{s.as} CH₂, CH₃
- **MS**: m/z: 399(M⁺.), 313, 297, 265, 247, 220, 111, 97, 95, 83, 69, 57, 55

| | | |
|---|---|---|
| Siedepunkt: 170-174°C/1·10⁻¹ mbar | | |
| **Elementaranalyse:** | C_{gemessen}: 72,4 % | C_{berechnet}: 72,3 % |
| | H_{gemessen}: 12,0 % | H_{berechnet}: 11,6 % |

### Beispiele 9 und 10:

Die Beispiele 9 und 10 illustrieren einen Vergleich der Katalysatoren Nafion SAC 13 (A) und Amberlyst 15 (B) bei der Addition von Pivalinsäure an die Doppelbindung von Ölsäuremethylester. Die Reaktionsparameter waren: Katalysatorbelastung 4 mmol/g Katalysator, molarer Überschuss Pivalinsäure im Verhältnis zu Ölsäuremethylester = 25, Temperatur 120°C, Reaktionszeit 8 h, Batchreaktion unter Normaldruck; Einsatz der Katalysatoren ohne vorherige Trocknung.

| Beispiel | Katalysator | Umsatz Ölsäuremethylester [%] | Selektivität zu Verbindung III [%] | Ausbeute an Verbindung III [%] |
|---|---|---|---|---|
| 9 | A | 32 | 98 | 31 |
| 10 | B | 22 | 50 | 11 |

### Beispiele 11 bis 13

Die Beispiele 11 bis 13 zeigen einen Vergleich verschiedener Y-Zeolithe als heterogene Katalysatoren bei der Addition von Pivalinsäure an die Doppelbindung von Ölsäuremethylester. Die Reaktionsparameter waren Katalysatorbelastung 4 mmol/g Katalysator, molarer Überschuss Pivalinsäure im Verhältnis zu Ölsäuremethylester = 25, Temperatur 120°C, Reaktionszeit 48h, Batchreaktion unter Normaldruck; Einsatz der Katalysatoren nach vorheriger Kalzinierung bei 550°C über 12 Stunden.

| Katalysatornummer | Hersteller und Bezeichnung | Modul: SiO₂/Al₂O₃ |
|---|---|---|
| 7 | Valor CBV-780 | 22 |
| 9 | CBV-780SDUS | 28 |
| 32 | CBU-780 | 24 |

| Beispiel | Katalysator | Umsatz Ölsäuremethylester | Selektivität zu Verbindung III [%] | Ausbeute an Verbindung III [%] |
|---|---|---|---|---|
| 11 | 7 | 45,6 | 91,0 | 41,5 |
| 12 | 9 | 33,5 | 92,2 | 30,9 |
| 13 | 32 | 34,3 | 93 | 31,9 |

### Beispiele 14 und 15:

Das Beispiel 14 zeigt die Reproduzierbarkeit der Ergebnisse mit dem Y-Zeolith, der dem Katalysator 7 entspricht. Im Beispiel 15 konnte gezeigt werden, dass der Y-Zeolith nach einer Kalzinierung wiederholt eingesetzt werden kann, ohne, wie beim Katalysator 32 zu sehen, an Aktivität zu verlieren. In beiden Beispielen wurde wie in den Beispielen 11 bis 13 die Addition von Pivalinsäure an die Doppelbindung von Ölsäuremethylester durchgeführt. Die Reaktionsparameter waren: Katalysatorbelastung 4 mmol/g Katalysator, molarer Überschuss Pivalinsäure im Verhältnis zu Ölsäuremethylester = 25, Temperatur 120°C, Reaktionszeit: 48 h, Batchreaktion unter Normaldruck; Einsatz der Katalysatoren nach vorheriger Kalizinierung bei 550°C über 12 Stunden.

| Beispiel | Katalysator | Umsatz Ölsäuremethylester | Selektivität zu Verbindung III [%] | Ausbeute an Verbindung III [%] |
|---|---|---|---|---|
| 14 | 7 | 46,7 | 91,9 | 42,9 |
| 15 | 32 | 40,4 | 90,8 | 36,7 |

### Beispiele 16 bis 18:

Beispiele 16 bis 18 illustrieren die Auswirkung eines höheren Verzweigungsgrades der addierten Carbonsäure auf Umsatz und Selektivität. Einsatzstoffe waren Ölsäuremethylester, Katalysator Nafion SAC 13, molarer Überschuss Carbonsäure im Verhätnis zu Ölsäuremethylester = 50 (Pivalinsäure = 25), Temperatur 120°C, Reaktionszeit 8 h, Batchreaktion unter Normaldruck; Einsatz des Katalysators ohne vorherige Trocknung.

| Beispiel | Eingesetzte Carbonsäure | Umsatz Ölsäuremethylester [%] | Selektivität zum Stearinsäureaddukt [%] | Ausbeute an Stearinsäureaddukt [%] |
|---|---|---|---|---|
| 16 | Ameisensäure | 83 | 77 | 64 |
| 17 | Essigsäure | 47 | 83 | 39 |
| 18 | Pivalinsäure | 32 | 98 | 31 |

### Beispiele 19 und 20

Die Beispiele 19 und 20 zeigen das Scale up der Addition von Pivalinsäure an die Doppelbindung von Ölsäuremethylester. Katalysator war Nafion SAC 13, die Reaktionsparameter waren: Katalysatorbelastung 4 mmol/g Katalysator, molarer Überschuss Pivalinsäure im Verhätnis zu Olsäuremethylester = 25, Temperatur 120°C, Batchreaktion unter Normaldruck; Einsatz der Katalysatoren ohne vorherige Trocknung.

| Beispiel | eingesetzte Menge Ölsäuremethylester [g] | Reaktionszeit [h] | Umsatz Ölsäuremethylester [%] | Selektivität zu Verbindung III [%] | Ausbeute an Verbindung III [%] |
|---|---|---|---|---|---|
| 19 | 1 | 8 | 29 | 88 | 26 |
| 20 | 100 | 15 | 32 | 98 | 31 |

### Beispiele 21 bis 25

Die Beispiele 21 bis 25 illustrieren das Verhalten des Reaktionssystems bei Vorbehandlung, Wiedereinsatz und Regenerierung von Nafion SAC 13 in der Addition von Pivalinsäure an die Doppelbindung von Ölsäuremethylester. Die Reaktionsbedingungen waren: Katalysatorbelastung 4 mmol/g Katalysator, molarer Überschuss Pivalinsäure im Verhätnis zu Ölsäuremethylester = 25, Temperatur 120°C, Batchreaktion unter Normaldruck, Reaktionszeit 15 h.

| Beispiel | Katalysatorbehandlung | Umsatz Ölsäuremethylester [%] | Selektivität zu Verbindung III [%] | Ausbeute an Verbindung III [%] |
|---|---|---|---|---|
| 21 | unbehandelt | 29 | 88 | 26 |
| 22 | unter HV bei 20°C 24 h getrocknet | 45 | 96 | 44 |
| 23 | 2. Einsatz, mit Aceton gespült und getrocknet | 36 | 89 | 32 |
| 24 | 3. Einsatz, mit Aceton gespült und getrocknet | 33 | 97 | 32 |
| 25 | gebraucht, mit Aceton gespült und mit HCl regeneriert, getrocknet | 41 | 91 | 38 |

### Beispiel 26

In einem weiteren Experiment wurde 3-Benzyloxy-2,2-dimethylpropionsäure als Säurekomponente eingesetzt.

### 3-Benzyloxy-2,2-dimethylpropionsäure

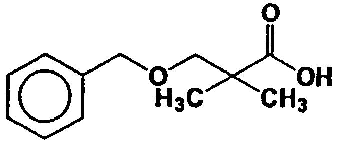

Die spektroskopischen Daten der Verbindung sind weiter vorne im Text bereits angegeben.
Es wurde Nafion SAC 13 als Katalysator für die Acyloxylierung von Ölsäuremethylester verwendet. Es wurden folgende Reaktionsbedingungen gewählt: Katalysatorbelastung 4 mmol/g Katalysator, molarer Überschuss Pivalinsäure im Verhätnis zu Ölsäuremethylester = 25, Temperatur 120°C, Batchreaktion unter Normaldruck, Reaktionszeit 24 h.

| Beispiel | Katalysatorbehandlung | Umsatz Ölsäuremethylester [%] | Selektivität zur Zielverbindung [%] | Ausbeute an Zielverbindung [%] |
|---|---|---|---|---|
| 26 | unter HV bei 20°C 24 h getrocknet | 26,5 | 36,3 | 9,6 |

### Beispiel 27:

Beispiel 27 illustriert die Eigenschaften der nach den Beispiel 1 und 4 hergestellten Ester Formyloxystearinsäuremethylester und [2,2-Dimethylpropionyloxy]-stearinsäuremethylester im Vergleich zu dem reinen Ölsäuremethylester (Basisrohstoff).

| | Ölsäuremethylester | Formyloxystearinsäuremethylester | [2,2-Dimethylpropionyltoxy] stearinsäuremethylester |
|---|---|---|---|
| KV (40°C) [mm²/s] | 4,5 | 12,0 | 13,1 |
| KV (100°C) [mm²/s] | 1,7 | 3,1 | 3,4 |
| VI | 178 | 120 | 139 |
| ROBOT ¹⁾ [min] | 10 | 165 | 27 |
| Pourpoint [°C] | -18 | -18 | -9 |
| Siedepunkt [°C] | 133 /10⁻¹mbar | 160-174 /10⁻¹mbar | 170-174 /10⁻¹mbar |
| Dichte (15°C) [kg/m³] | | 933,65 | 902,73 |

| | | | |
|---|---|---|---|
| ¹⁾ Bedingungen: 35 g Schmierstoff; 6,25 bar Sauerstoff; T = 120°C; ohne Katalysator | | | |

### Weitere Beispiele:

Als weitere verzweigte Carbonsäuren zur Addition an die Doppelbindungen natürlicher Fettsäuren und natürlicher Fettsäurederivate kommen z. B. die nachstehenden Carbonsäuren in Betracht, deren spektroskopische Eigenschaften vorstehend in der Beschreibung im einzelnen bereits angegeben sind:

### 3-Propyloxy-2,2-dimethylprpionsäure

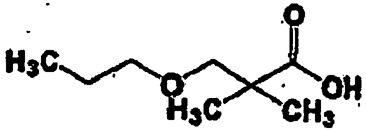

### 3-Butyloxy-2,2-dimethylpropionsäure

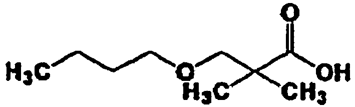

### 3-Isobutyloxy-2,2-dimethylpropionsäure

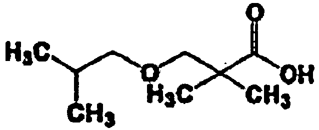

### 3-(2-Ethylbutyloxy)-2,2-dimethylpropionsäure

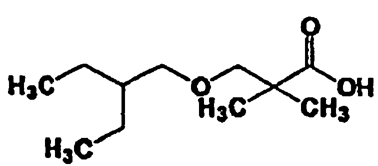

und

### 3-(2-Methylbenzyloxy)-2,2-dimethylpropionsäure

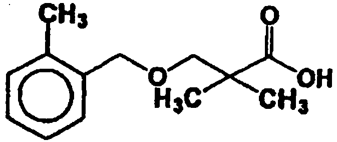

## Patentansprüche

1. Verwendung eines Reaktionsproduktes erhältlich durch Acyloxylierung zumindest einer Doppelbindung von Fettsäuren und/oder Fettsäurederivaten und deren Gemischen in oder als Schmierstoff und/oder Druckübertragungsmittel, **dadurch gekennzeichnet, dass** die Doppelbindung acyloxyliert ist durch Reste von Neocarbonsäuren mit der allgemeinen chemischen Formel: 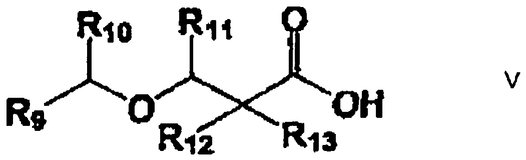 in der R₉, R₁₀, R₁₂ und/oder R₁₃ gleich oder verschieden sind und Wasserstoff, geradkettige oder verzweigte Alkyl-, Alkenyl, oder Alkinylreste mit bis zu 18 Kohlenstoffatomen, Cycloalkyl- oder Cycloalkenylreste mit 5 bis 8 Kohlenstoffatomen, Aryl-, Aralkyl- oder Alkenylarylreste mit 6 bis 16 Kohlenstoffatomen oder heterocyclische Reste bedeuten,
wobei jeweils die Reste R₉ und R₁₀ und/oder R₁₂ und R₁₃ zusammen mit dem Kohlenstoffatom, an das sie gebunden sind, ein Cycloalkan, Cycloalken oder einen Heterocyclus mit 5 bis 7 Ringgliedern bilden können, R₉, R₁₀, R₁₂ und R₁₃ überdies Substituenten tragen können und R₁₁ für Wasserstoff oder einen geradkettigen oder verzweigten Alkylrest steht.

2. Verwendung gemäß Anspruch 1, **dadurch gekennzeichnet, dass** unter die allgemeine chemischen Formel V Neocarbonsäuren fallen, in denen R₉, R₁₀, R₁₂ und R₁₃ gleich oder verschieden sind und Wasserstoff, geradkettige oder verzweigte Alkylreste mit 1 bis 12, insbesondere 1 bis 6 Kohlenstoffatomen, Alkenyl- oder Alkinylreste mit 2 bis 12, insbesondere 2 bis 6 Kohlenstoffatomen bedeuten, oder in denen R₉, R₁₀, R₁₂ und R₁₃ für Cycloalkyl- oder Cycloalkenylreste mit 5 oder 6 Kohlenstoffatomen, für Aryl-, Alkylaryl-, Aralkyl- oder Alkenylarylreste mit 6 bis 12 Kohlenstoffatomen und für heterocyclische Reste, die ein oder mehrere Stickstoff- und/oder Sauerstoff- und/oder Schwefelatome enthalten, stehen und in denen R₁₁ Wasserstoff oder ein geradkettiger oder verzweigter Alkylrest mit 1 bis 12, insbesondere 1 bis 8, vorzugsweise 1 bis 4 Kohlenstoffatomen ist, bevorzugt Wasserstoff,

3. Verwendung gemäß Anspruch 1, **dadurch gekennzeichnet, dass** die Fettsäuren und/oder Fettsäurederivate Ölsäure, Linolsäure, Linolensäure, Palmitoleinsäure, Eicosensäure, Erucasäure oder Gemische von diesen oder Ölsäuremethylester, Linolsäuremethylester, Linolensäuremethylester, Palmitoleinsäuremethylester, Glycerintrioleat, Glycerintrilinolat, Glycerintrilinoleat, Trimethylolpropantrioleat, Trimethylolpropantrilinolat, Trimethylolpropantrilinoleat, Pentaerythrittetraoleat, Pentaerythrittetralinolat, Pentaerythrittetralinoleat oder Gemische von diesen oder nachwachsende Rohstoffe wie Rapsöl, Sonnenblumenöl, Palmöl, Kokosöl oder Olivenöl sind oder enthalten.

4. Verwendung nach einem der Ansprüche 1 bis 3 in reiner Form oder als Schmierstoffadditiv, insbesondere als Funktionsflüssigkeit in Hydraulikölen oder für Tribosysteme.

5. An einer Doppelbindung acyloxylierte Fettsäure oder Fettsäurederivat
a) der allgemeinen Formel: 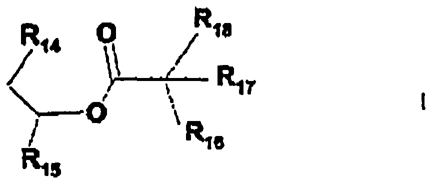 mit
R₁₄ ein Carbonsäurereste R-COOH mit R = Alkyl-, Alkenyl-, Dienyl- oder Polyenylrest mit 2 bis 20 C-Atomen und 0 bis 4 Doppelbindungen, vorzugsweise mit 3 bis 11 C - Atomen und 0 bis 2 Doppelbindungen, oder deren Ester mit Monoalkoholen, Diolen oder Polyolen;
R₁₅ Alkyl-, Alkenyl-, Dienyl- oder Polyenylrest mit 2 bis 20 C - Atomen und 0 bis 4 Doppelbindungen, vorzugsweise 3 bis 14 C-Atomen und 0 bis 3 Doppelbindungen, insbesondere 3 bis 10 C-Atomen und 0 bis 2 Doppelbindungen, ist,
R₁₆ H oder ein Alkylrest mit 1 bis 10 C-Atomen, Arylrest mit Alkyl- und anderen Substituenten am aromatischen Ring, Aralkylrest, Alkyloxyreste mit 1 bis 10 C-Atomen und Phosphor oder Schwefel als weiteren Heteroatomen, Aralkyloxyrest mit Alkyl- und heteroatomhaltigen Substituenten am aromatischen Ring ist,
R₁₇ ein Alkylrest mit 1 bis 10 C-Atomen, Arylrest mit Alkyl- und anderen Substituenten am aromatischen Ring, Aralkylrest, Alkyloxyreste mit 1 bis 10 C-Atomen und Phosphor oder Schwefel als weiteren Heteroatomen, Aralkyloxyrest mit Alkyl- und heteroatomhaltigen Substituenten am aromatischen Ring ist,
R₁₈ ein Alkylrest mit 1 bis 10 C-Atomen, Arylrest mit Alkyl- und anderen Substituenten am aromatischen Ring, Aralkylrest, Alkyloxyreste mit 1 bis 10 C-Atomen und Phosphor oder Schwefel als weiteren Heteroatomen, Aralkyloxyrest mit Alkyl- und heteroatomhaltigen Substituenten am aromatischen Ring ist,
wobei R₁₆, R₁₇, R₁₈ gleich oder verschieden und im Falle von R₁₆ = H, R₁₇ und R₁₈ gleich oder verschieden sein können,
oder
b) erhältlich durch Acyloxylierung zumindest einer Doppelbindung von Fettsäuren und/oder Fettsäurederivaten und deren Gemischen mit: 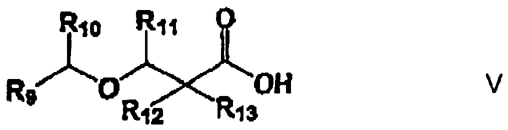 in der R₉, R₁₀, R₁₂ und/oder R₁₃ gleich oder verschieden sind und Wasserstoff, geradkettige oder verzweigte Alkyl-, Alkenyl, oder Alkinylreste mit bis zu 18 Kohlenstoffatomen, Cycloalkyl- oder Cycloalkenylreste mit 5 bis 8 Kohlenstoffatomen, Aryl-, Aralkyl- oder Alkenylarylreste mit 6 bis 16 Kohlenstoffatomen oder heterocyclische Reste bedeuten,
wobei jeweils die Reste R₉ und R₁₀ und/oder R₁₂ und R₁₃ zusammen mit dem Kohlenstoffatom, an das sie gebunden sind, ein Cycloalkan, Cycloalken oder einen Heterocyclus mit 5 bis 7 Ringgliedern bilden können, R₉, R₁₀, R₁₂ und R₁₃ überdies Substituenten tragen können und R₁₁ für Wasserstoff oder einen geradkettigen oder verzweigten Alkylrest steht.
oder
c) erhältlich durch Acyloxylierung zumindest einer Doppelbindung von Fettsäuren und/oder Fettsäurederivaten und deren Gemischen mit:
- 3-Propyloxy-2,2-dimethylpropionsäure,
- 3-Butyloxy-2,2-dimethylpropionsäure,
- 3-Isobutyloxy-2,2-dimethylpropionsäure,
- 3-(2-Ethylbutyloxy)-2,2-dimethylpropionsäure,
- 3-Benzyloxy-2,2-dimethylpropionsäure und/oder
- 3-(2-Methylbenzyloxy)-2,2-dimethylpropionsäure.

6. Verfahren zur Herstellung von an mindestens einer Doppelbindung acyloxylierter Fettsäure oder Fettsäurederivate durch Acyloxylierung mit Carbonsäuren, **dadurch gekennzeichnet, dass** die Carbonsäuren Neocarbonsäuren sind, insbesondere ausgewählt aus der folgenden Gruppe:
- 3-Propyloxy-2,2-dimethylpropionsäure,
- 3-Butyloxy-2,2-dimethylpropionsäure,
- 3-Isobutyloxy-2,2-dimethylpropionsäure,
- 3-(2-Ethylbutyloxy)-2,2-dimethylpropionsäure,
- 3-Benzyloxy-2,2-dimethylpropionsäure und/oder
- 3-(2-Methylbenzyloxy)-2,2-dimethylpropionsäure.

## Claims

1. Use of a reaction product obtainable by acyloxylating at least one double bond of fatty acids and/or fatty acid derivatives and the mixtures thereof in or as a lubricant and/or pressure transfer medium, **characterized in that** the double bond is acyloxylated by moieties of neocarboxylic acids of the general chemical formula: 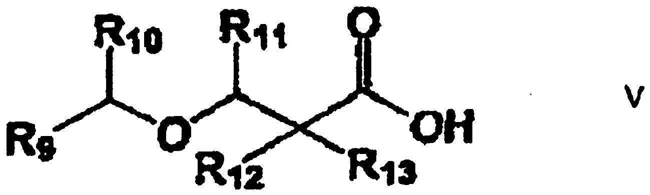 in which R₉, R₁₀, R₁₂ and/or R₁₃ are the same or different and denote hydrogen, straight-chain or branched alkyl, alkenyl or alkinyl moieties having up to 18 carbon atoms, cycloalkyl or cycloalkenyl moieties having 5 to 8 carbon atoms, aryl, aralkyl or alkenyl aryl moieties having 6 to 16 carbon atoms or heterocyclic moieties, wherein each of the moieties R₉ and R₁₀ and/or R₁₂ and R₁₃, together with the carbon atom to which they are bound, can form a cycloalkane, cycloalkene or a heterocycle having 5 to 7 ring members, R₉, R₁₀, R₁₂ and R₁₃ moreover can carry substituents and R₁₁ denotes hydrogen or a straight-chain or branched alkyl moiety.

2. Use according to claim 1, **characterized in that** the general chemical formula V covers neocarboxylic acids in which R₉, R₁₀, R₁₂ and/or R₁₃ are the same or different and denote hydrogen, straight-chain or branched alkyl moieties having 1 to 12, and more particularly 1 to 6, carbon atoms, alkenyl or alkinyl moieties having 2 to 12, and more particularly 2 to 6, carbon atoms, or in which R₉, R₁₀, R₁₂ and R₁₃ denote cycloalkyl or cycloalkenyl moieties having 5 or 6 carbon atoms, or they denote aryl, alkylaryl, aralkyl or alkenyl aryl moieties having 6 to 12 carbon atoms and denote heterocylic moieties which contain one or more nitrogen and/or oxygen and/or sulfur atoms, and in which R₁₁ denotes hydrogen or a straight-chain or branched alkyl moiety having 1 to 12, particularly 1 to 8, and preferably 1 to 4, carbon atoms, preferably hydrogen.

3. Use according to claim 1, **characterized in that** the fatty acids and/or fatty acid derivatives are or contain oleic acid, linoleic acid, linolenic acid, palmitoleic acid, eicosenoic acid, erucic acid or mixtures thereor, or oleic acid methyl ester, linoleic acid methyl ester, linolenic acid methyl ester, palmitoleic acid methyl ester, glycerin trioleate, glycerin trilinolate, glycerin trilinoleate, trimethylol propane trioleate, trimethylol propane trilinilonate, trimethylol propane trilinoleate, pentaerythrite tetraoleate, pentaerythrite tetralinolate, pentaerythrite tetralinoleate or mixtures thereof, or renewable resources such as rapeseed oil, sunflower oil, palm oil, coconut oil or olive oil.

4. Use according to any one of claims 1 to 3 in pure form or as a lubricant additive, in particular as a functional liquid in hydraulic fluids or for tribological systems.

5. A fatty acid or fatty acid derivative which is acyloxylated on a double bond and
a) has the general formula: 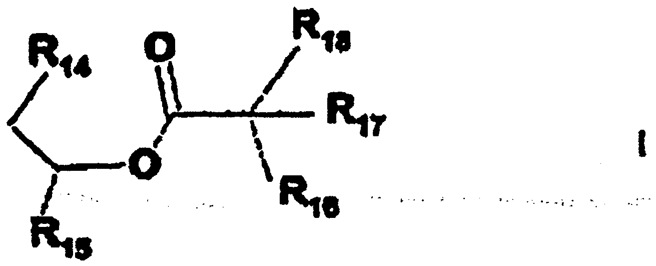 wherein
R₁₄ is a carboxylic acid moiety R-COOH with R = alkyl, alkenyl, dienyl or polenyl moiety having 2 to 20 carbon atoms and 0 to 4 double bonds, preferably having 3 to 11 carbon atoms and 0 to 2 double bonds, or the esters thereof with monoalcohols, diols or polyols;
R₁₅ is an alkyl, alkenyl, dienyl or polyenyl moiety having 2 to 20 carbon atoms and 0 to 4 double bonds, preferably having 3 to 14 carbon atoms and 0 to 3 double bonds, and more particularly 3 to 10 carbon atoms and 0 to 2 double bonds;
R₁₆ is H or an alkyl moiety having 1 to 10 carbon atoms, an aryl moiety with alkyl and other substituents on the aromatic ring, an aralkyl moiety, alkyloxy moieties having 1 to 10 carbon atoms and phosphorus or sulfur as further heteroatoms, aralkyloxy moiety with alkyl- and heteroatom-containing substituents on the aromatic ring,
R₁₇ is an alkyl moiety having 1 to 10 carbon atoms, an aryl moiety with alkyl and other substituents on the aromatic ring, aralkyl moiety, alkyloxy moieties having 1 to 10 carbon atoms and phosphorus or sulfur as further heteroatoms, aralkyloxy moiety with alkyl- and heteroatom-containing substituents on the aromatic ring;
R₁₈ is an alkyl moiety having 1 to 10 carbon atoms, an aryl moiety with alkyl and other substituents on the aromatic ring, aralkyl moiety, alkyloxy moieties having 1 to 10 carbon atoms and phosphorus or sulfur as further heteroatoms, aralkyloxy moiety with alkyl- and heteroatom-containing substituents on the aromatic ring;
where R₁₆, R₁₇, R₁₈ can be the same or different and in the case of R₁₆ = H, R₁₇ and R₁₈ can be the same or different, or
b) obtainable by acyloxylating at least one double bond of fatty acids and/or fatty acid derivatives and the mixtures thereof with: 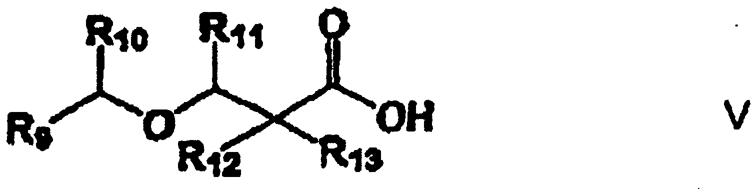 in which R₉, R₁₀, R₁₂ and/or R₁₃ are the same or different and denote hydrogen, straight-chain or branched alkyl, alkenyl or alkinyl moieties having up to 18 carbon atoms, cycloalkyl or cycloalkenyl moieties having 5 to 8 carbon atoms, aryl, aralkyl or alkenyl aryl moieties having 6 to 16 carbon atoms or heterocyclic moieties, wherein each of the moieties R₉ and R₁₀ and/or R₁₂ and R₁₃, together with the carbon atom to which they are bound, can form a cycloalkane, cycloalkene or a heterocycle having 5 to 7 ring members, R₉, R₁₀, R₁₂ and R₁₃ moreover can carry substituents and R₁₁ denotes hydrogen or a straight-chain or branched alkyl moiety, or
c) obtainable by acyloxylating at least one double bond of fatty acids and/or fatty acid derivatives and the mixtures thereof with:
- 3-propyloxy-2,2-dimethylpropionic acid,
- 3-butyloxy-2,2-dimethylpropionic acid,
- 3-isobutyloxy-2,2-dimethylpropionic acid,
- 3-(2-ethylbutyloxy)-2,2-dimethylpropionic acid,
- 3-benzyloxy-2,2-dimethylpropionic acid, and/or
- 3-(2-methylbenzyloxy)-2,2-dimethylpropionic acid.

6. A method for producing fatty acid or fatty acid derivatives acyloxylated on at least one double bond by acyloxylation with carboxylic acids, **characterized in that** the carboxylic acids are neocarboxylic acids, in particular selected from the following group:
- 3-propyloxy-2,2-dimethylpropionic acid,
- 3-butyloxy-2,2-dimethylpropionic acid,
- 3-isobutyloxy-2,2-dimethylpropionic acid,
- 3-(2-ethylbutyloxy)-2,2-dimethylpropionic acid,
- 3-benzyloxy-2,2-dimethylpropionic acid, and/or
- 3-(2-methylbenzyloxy)-2,2-dimethylpropionic acid.

## Revendications

1. Utilisation d'un produit réactionnel pouvant être obtenu par acyloxylation d'au moins une double liaison d'acides gras et/ou de dérivés d'acides gras et de leurs mélanges dans des lubrifiants et/ou des agents de transfert de pression ou comme tels, **caractérisée en ce que** la double liaison est acyloxylée par des radicaux d'acides néocarboxyliques ayant la formule chimique générale : 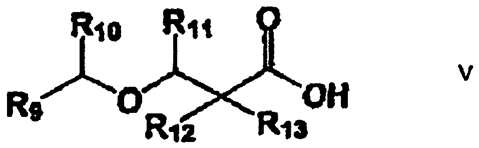 dans laquelle R₉, R₁₀, R₁₂ et/ou R₁₃ sont identiques ou différents et représentent un atome d'hydrogène, des radicaux alkyle linéaire ou ramifié, alcényle ou alcinyle ayant jusqu'à 18 atomes de carbone, des radicaux cycloalkyle ou cycloalcényle ayant de 5 à 8 atomes de carbone, des radicaux aryle, aralkyle ou alcénylaryle ayant de 6 à 16 atomes de carbone ou des radicaux hétérocycliques,
où respectivement les radicaux R₉ et R₁₀ et/ou R₁₂ et R₁₃ peuvent constituer, conjointement avec l'atome de carbone, auquel ils sont reliés, un cycloalcane, un cycloalcène ou un hétérocycle ayant de 5 à 7 membres dans le cycle, R₉, R₁₀, R₁₂ et R₁₃ peuvent en outre porter des substituants et R¹¹ représente un atome d'hydrogène ou un radical alkyle linéaire ou ramifié.

2. Utilisation selon la revendication 1, **caractérisée en ce que** la formule chimique générale V englobe des acides néocarboxyliques, dans lesquels R₉, R₁₀, R₁₂ et R₁₃ sont identiques ou différents et représentent un atome d'hydrogène, des radicaux alkyle linéaire ou ramifié ayant de 1 à 12, de préférence de 1 à 6 atomes de carbone, des radicaux alcényle ou alcinyle ayant de 2 à 12, en particulier de 2 à 6 atomes de carbone, ou dans lesquels R₉, R₁₀, R₁₂ et R₁₃ représentent des radicaux cycloalkyle ou cycloalcényle ayant 5 ou 6 atomes de carbone, des radicaux aryle, alkylaryle, aralkyle ou alcénylaryle ayant de 6 à 12 atomes de carbone et des radicaux hétérocycliques, qui comprennent un ou plusieurs atomes d'azote et/ou d'oxygène et/ou de soufre, et dans lesquels R₁₁ représente un atome d'hydrogène, ou un radical alkyle linéaire ou ramifié ayant de 1 à 12, en particulier de 1 à 8, de préférence de 1 à 4 atomes de carbone, de préférence un atome d'hydrogène.

3. Utilisation selon la revendication 1, **caractérisée en ce que** les acides gras et/ou dérivés d'acide gras sont ou comprennent l'acide oléique, l'acide linoléique, l'acide linolénique, l'acide palmitoléique, l'acide éicosanoïque, l'acide érucique ou des mélanges de ceux-ci ou l'ester méthylique d'acide oléique, l'ester méthylique d'acide linoléique, l'ester méthylique d'acide linolénique, l'ester méthylique d'acide palmitoléique, le trioléate de glycérine, le trilinolate de glycérine, le trilinoléate de glycérine, le trioléate de triméthylolpropane, le trilinolate de triméthylolpropane, le trilinoléate de triméthylolpropane, le tétraoléate de pentaérythrite, le tétralinolate de pentaérythrite, le tétralinoléate de triméthylolpropane ou des mélanges de ceux-ci ou les matières premières dont ils sont dérivés comme l'huile de colza, l'huile de tournesol, l'huile de palme, l'huile de coco ou l'huile d'olive.

4. Utilisation selon l'une des revendications 1 à 3 sous une forme pure ou sous forme d'additif de lubrifiant, en particulier de fluide fonctionnel dans les huiles hydrauliques ou pour les systèmes tribologiques.

5. Acide gras ou dérivé d'acide gras acyloxylé sur une double liaison
a) de formule générale : 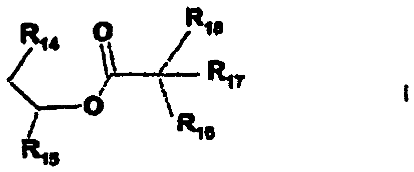 avec
R¹⁴ représentant un radical d'acide carboxylique R-COOH avec R = radical alkyle, alcényle, diényle ou polyényle ayant de 2 à 20 atomes de C et 0 à 4 doubles liaisons, de préférence de 3 à 11 atomes de C et 0 à 2 doubles liaisons, ou son ester avec des monoalcools, des diols, ou des polyols ;
R¹⁵ représentant un radical alkyle, alcényle, diényle ou polyényle ayant de 2 à 20 atomes de C et 0 à 4 doubles liaisons, de préférence de 3 à 14 atomes de C et 0 à 3 doubles liaisons, de préférence de 3 à 10 atomes de C et 0 à 2 doubles liaisons,
R¹⁶ représentant H ou un radical alkyle ayant de 1 à 10 atomes de C, un radical aryle ayant des substituants alkyle et d'autres substituants sur le cycle aromatique, un radical aralkyle, des radicaux alkyloxy ayant de 1 à 10 atomes de C et du phosphore ou du soufre comme autres hétéroatomes, un radical aralkyloxy ayant des substituants alkyle et des substituants comprenant un hétéroatome sur le cycle aromatique,
R¹⁷ représentant un radical alkyle ayant de 1 à 10 atomes de C, un radical aryle ayant des substituants alkyle et d'autres substituants sur le cycle aromatique, un radical aralkyle, des radicaux alkyloxy ayant de 1 à 10 atomes de C et du phosphore ou du soufre comme autres hétéroatomes, un radical aralkyloxy ayant des substituants alkyle et des substituants comprenant un hétéroatome sur le cycle aromatique,
R¹⁸ représentant un radical alkyle ayant de 1 à 10 atomes de C, un radical aryle ayant des substituants alkyle et d'autres substituants sur le cycle aromatique, un radical aralkyle, des radicaux alkyloxy ayant de 1 à 10 atomes de C et du phosphore ou du soufre comme autres hétéroatomes, un radical aralkyloxy ayant des substituants alkyle et des substituants comprenant un hétéroatome sur le cycle aromatique,
où R₁₆, R₁₇, R₁₈ peuvent être identiques ou différents et dans le cas où R₁₆ = H, R₁₇ et R₁₈ peuvent être identiques ou différents,
ou
b) pouvant être obtenu par acyloxylation d'au moins une double liaison d'acides gras et/ou de dérivés d'acide gras et de leurs mélanges avec : 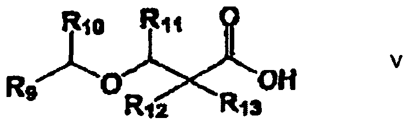 dans laquelle R₉, R₁₀, R₁₂ et/ou R₁₃ sont identiques ou différents et représentent un atome d'hydrogène, des radicaux alkyle linéaire ou ramifié, alcényle ou alcinyle ayant jusqu'à 18 atomes de carbone, des radicaux cycloalkyle ou cycloalcényle ayant de 5 à 8 atomes de carbone, des radicaux aryle, aralkyle ou alcénylaryle ayant de 6 à 16 atomes de carbone ou des radicaux hétérocycliques,
où respectivement les radicaux R₉ et R₁₀ et/ou R₁₂ et R₁₃ peuvent former, conjointement avec l'atome de carbone auquel ils sont reliés, un cycloalcane, un cycloalcène ou un hétérocycle ayant de 5 à 7 membres, R₉, R₁₀, R₁₂ et R₁₃ peuvent porter en outre des substituants et R₁₁ représente un atome d'hydrogène ou un radical alkyle linéaire ou ramifié,
ou
c) pouvant être obtenu par acyloxylation d'au moins une double liaison d'acides gras et/ou de dérivés d'acide gras et de leurs mélanges avec :
- l'acide 3-propyloxy-2,2-diméthylpropionique,
- l'acide 3-butyloxy-2,2-diméthylpropionique,
- l'acide 3-isobutyloxy-2,2-diméthylpropionique,
- l'acide 3-(2-éthylbutyloxy)-2,2-diméthylpropionique,
- l'acide 3-benzyloxy-2,2-diméthylpropionique et/ou
- l'acide 3-(2-méthylbenzyloxy)-2,2-diméthylpropionique.

6. Procédé de production d'acides gras ou de dérivés d'acides gras acyloxylés sur au moins une double liaison par acyloxylation avec des acides carboxyliques, **caractérisé en ce que** les acides carboxyliques sont des acides néocarboxyliques, en particulier choisis dans le groupe suivant constitué par :
- l'acide 3-propyloxy-2,2-diméthylpropionique,
- l'acide 3-butyloxy-2,2-diméthylpropionique,
- l'acide 3-isobutyloxy-2,2-diméthylpropionique,
- l'acide 3-(2-éthylbutyloxy)-2,2-diméthylpropionique,
- l'acide 3-benzyloxy-2,2-diméthylpropionique et/ou
- l'acide 3-(2-méthylbenzyloxy)-2,2-diméthylpropionique.
